# EUROPEAN PATENT APPLICATION

(11) **EP 1 518 501 A2**
(43) Date of publication of application: **30.03.2005**
(21) Application number: 04254916.2
(22) Date of filing: 16.08.2004
(51) Int. Cl.: A61B 17/02, A61B 17/15

(54) **Knee surgery tensor and alignment device, and computer, program, and method.**

(30) Priority: 15.08.2003 GB 0319257
(71) Applicant: Finsbury (Development) Limited, Leatherhead, Surrey KT22 0BA (GB)
(72) Inventor: Freeman, Michael, London NW2 7LX (GB)
(74) Representative: Smaggasgale, Gillian Helen

(57) **Abstract**

The invention relates to surgical instruments, a computer program element which provides control or information relating to the surgical instruments, a computer program providing the same, a computer readable medium comprising the computer program, a method for implementing a computer-aided means of guiding a surgeon through a desired surgical procedure and to a computer aided surgical procedure. In a preferred embodiment, the invention relates to instruments, a computer program element, computer programs, a computer readable medium and methods for use in knee replacement operations.

## Description

The present invention relates to surgical instruments, a computer program element which provides control or information relating to the surgical instruments, a computer program providing the same, a computer readable medium comprising the computer program, a method for implementing a computer-aided means of guiding a surgeon through a desired surgical procedure and to a computer aided surgical procedure. In a preferred embodiment, the invention relates to instruments, a computer program element, computer programs, a computer readable medium and methods for use in knee replacement operations.

The human knee is a complex joint formed between the femur and the tibia. Although the axis of the tibia is substantially vertical in an erect standing human, the femur shaft axis is usually at an angle of about 7° out of the vertical. This is to allow the weight of the upper part of the body to be transmitted from the acetabulum of the hip joint to the ball head at the top of the femur, which is offset from the femur shaft axis, in a straight line from the centre of the hip to the centre of the knee and then via the tibia in the same straight line to the centre of the ankle. This straight line is known as the Maquet line.

At the lower end of the femur there are formed two femoral condyles, namely a lateral condyle and a medial condyle. Corresponding generally concave lateral and medial depressions are formed on the upper end of the tibia. A layer of cartilage is interposed between the load bearing surfaces of the joint. A pair of collateral ligaments connect the femur to the tibia, one on each side of the knee joint. These collateral ligaments play an essential role in holding the knee joint together and in providing stability therefor. Thus in the natural knee they are taut throughout the range of motion of the knee joint as the person bends his or her knee in walking or running. Moreover the line joining the points of attachment of the collateral ligaments to the femur effectively acts as the axis about which the knee bends.

A further pair of cruciate ligaments extend parallel to a substantially sagittal plane of the knee joint between the medial and lateral condyles. The anterior cruciate ligament connects an anterior part of the top end of the tibia to a posterior surface of a recess at the bottom end of the femur at the rear of the knee joint. The posterior cruciate ligament is positioned adjacent the anterior cruciate ligament and connects a posterior part of the top of the tibia to another part of the same back surface of the afore-mentioned recess in the rear of the knee joint. The patella covers the front side of the knee joint.

The human knee can bend through an angle of approximately 160°. In the flexed condition of the knee a person can rotate his or her tibia about its axis relative to the femur through a small angle. However, in the straightened condition of the knee, the tibia becomes effectively locked with respect to the femur so that rotation of the tibia about its axis relative to the femur becomes essentially impossible.

With passage of time many people suffer wear of, or damage to, the bearing surfaces of one or both of the bones of the knee joint. In severe cases it is expedient to perform a knee replacement operation in which the worn or damaged bone is resurfaced. In such an operation part of the upper end of the tibia and/or the lower end of the femur may be cut away and a tibial and/or femoral component fitted as an implant. If necessary, a patella implant can also be fitted in the course of the implantation operation.

An important consideration for a surgeon undertaking the implantation of a knee prosthesis is that the patient's soft tissues need to be correctly balanced in the two vitally important aspects required in successful total knee replacement. The first is to ensure that, at full extension of the knee, the medial and lateral tissues are correctly tensioned in an anatomically aligned knee. The second aspect is to ensure that the implants are chosen for size and positioned on the skeleton to ensure the correct balance of soft tissue tensions in both the extended and flexed knee.

There is a need for improved surgical instruments for use during orthopaedic operations, such as implantation of a knee prosthesis. More particularly there is a need for means for use during a knee replacement operation which will enable the surgeon to ensure that, at full extension of the knee, the medial and lateral tissues are correctly tensioned in an aligned knee. In addition there is a need to provide means which will enable a surgeon to select an implant having optimum dimensions and to implant this in optimal fashion in the patient's skeleton so as to ensure that there is the correct balance of soft tissue both in the extended knee and in the flexed knee.

The invention accordingly seeks to provide improved surgical instruments which will enable a surgeon to ensure that, at full extension of the knee, the medial and lateral tissues are correctly tensioned in an aligned knee. In addition the invention seeks to facilitate surgical operations by improved use of computer technology. It further seeks to provide a computer program which will, with the aid of appropriately designed surgical instruments, guide a surgeon through a surgical procedure and facilitate the keeping of records of the surgical procedure carried out on a patient. It is a further objective of the invention to provide means which will enable a surgeon to select, with the aid of a computer, an implant having optimum dimensions and to implant this in optimal fashion in the patient's skeleton so as to ensure that there is the correct balance of soft tissue both in the extended knee and in the flexed knee.

According to the first aspect of the present invention there is provided a tensor for use during the surgical implantation of a knee prosthesis including a tibial component and at least one femoral component, the tensor comprising:
a lower paddle having an under surface for contacting an upper surface of a resected tibia of the patient;
a first upper paddle movable towards and away from the lower paddle and having an upper surface for contacting a first one of the condyles of a corresponding femur of the patient;
a second upper paddle movable towards and away from the lower paddle and having an upper surface for contacting the other one of the condyles of the femur of the patient;
first adjustment means for moving the first upper paddle towards and away from the lower paddle;
second adjustment means for moving the second upper paddle towards and away from the lower paddle;
a first force sensor means associated with the first upper paddle which in use will measure a first force exerted by the first upper paddle against the first one of the condyles when the under surface of the lower paddle is in contact with the resected upper surface of the tibia of the patient and the first upper paddle is moved progressively away from the lower paddle into contact with the first one of the condyles;
a second load sensor means associated with the second upper paddle which in use will measure a second force exerted by the second upper paddle against the other one of the condyles when the under surface of the lower paddle is in contact with the resected upper surface of the tibia of the patient and the second upper paddle is moved progressively away from the lower paddle into contact with the other one of the condyles; and
signal output means for receiving signals from the first and second load sensor means indicative of the first and second forces respectively and for transmitting at least one output signal derived therefrom.

It will be understood that in use, the output signal will be transmitted to a suitable apparatus configured to receive the signal. The transmission may be by any suitable means either wireless or by a physical connection.

In a second embodiment of the present invention there is provided an alignment device for use during the surgical implantation of a knee prosthesis to aid in identifying the Maquet line, the device comprising:
first mounting means for location in the vicinity of a resected upper surface of a corresponding tibia of the patient;
a first pivot member mounted in the first mounting means so as to permit pivotal movement about a medial-lateral axis relative to the first mounting means;
detector means for detecting the magnitude of medial-lateral pivotal movement of the first pivot member;
second mounting means mounted to the pivot member;
elongate indicator means pivotally mounted at a lower end thereof in the second mounting means so as to permit pivotal movement about an anterior-posterior axis relative to the second mounting means, the elongate indicator means further having an upper end for positioning adjacent a hip of a patient; and
output means for producing an output signal from the detector means corresponding to the angular position of the first pivot member for transmitting said signal.

It will be understood that in use, the output signal will be transmitted to a suitable apparatus configured to receive the signal. The transmission may be by any suitable means either wireless or by a physical connection.

According to a third aspect of the present invention there is provided a computer program element comprising computer code means which when loaded on a computer will be adapted:
to receive output signals from at least one of a tensor of the above first aspect and an alignment device of the above second aspect;
to display information relating to the received signals; and
to receive input from a user.

The computer program element will preferably also compare information derived from the output signals with stored data such that information relating to corrections which may be required can be displayed. Thus, the computer element will enable the surgeon to be provided with suggestions as to the operation of the tensor and/or alignment device to achieve the optimum position.

In one arrangement, the computer program element will additionally allow the output signals and the input from the user to be recorded.

Additionally, the computer program element may record the time at which the output signals and the input from the user are received. Thus the program element may enable the procedure to be recorded for medical record purposes.

According to a fourth aspect of the present invention there is provided a computer program comprising the computer program element of the above third aspect. The computer program may also include means to enable the user to input additional notes. Thus the surgeons comments on the procedure may be recorded to supplement the medical records. These may be input by any suitable means either by recordal of voice or by input via a keyboard, tablet or the like. In one arrangement the input may be simply the input of a prompt to remind the surgeon to add the comment at a later time. The prompt may, for example, by input via a "click" from a foot pedal.

The computer program may additionally enable the display of information relating to the procedure to be carried out. Thus where the operation to be carried out is a knee replacement operation, information may be displayed describing, for example, the incision, the dislocation of the patella and the like. This may be provided as text, drawings, video and the like.

The computer program may be configured such that the user interface is provided as a plurality of screens. In this arrangement, each screen may include an interactive prompt to ask the user to confirm that the steps referred to on the screen had been successfully completed.

According to a fifth aspect of the present invention there is provided a computer readable medium having the computer program of the above fourth aspect recorded thereon. The computer readable medium may be in any suitable form.

According to a sixth aspect of the present invention there is provided a kit comprising the tensor of the above first aspect, the alignment guide of the above second aspect and the computer readable medium of the above fifth aspect. The kit preferable also includes instructions.

According to a sixth aspect of the present invention there is provided an automatic method of guiding a surgeon through a surgical procedure which includes a number of steps including at least one milestone step and at least one non-milestone step, the method comprising:
providing a computer having an output screen and an input device and loaded with a computer program arranged to display a plurality of screen displays on the output screen in response to input actions entered on the input device;
providing at least one surgical measuring instrument adapted to provide an output signal or signals representative of a measurement made by the surgeon following a respective milestone step of the surgical procedure and capable of interacting with the computer program; and
displaying on the output screen in turn a plurality of screen displays in succession in response to input actions entered on the input device, the plurality of screen displays including at least one milestone screen display corresponding to a respective milestone step of the surgical procedure;
wherein the computer program is arranged (i) to permit display of a next succeeding screen display on the output screen upon entry of a corresponding input action following completion by the surgeon of a non-milestone step of the surgical procedure and (ii) to block the display of the next screen display following completion by the surgeon of a milestone step unless the output signal or signals from the respective surgical measuring instrument corresponds or correspond to a respective predetermined value or values.

In such an automatic method the computer program can be arranged to record a value or values corresponding to an output signal or signals measured by the surgical measuring instrument at the completion of the or each milestone step of the surgical procedure. In addition, the computer program can be arranged to record a time at which at least one preselected input action is entered on the input device. Furthermore the computer program can be arranged to record one or more physiological characteristics of the patient during the course of the surgical procedure. In addition, the computer program can be arranged to record details of the patient prior to the commencement of the surgical procedure.

The input device may be any suitable device and may comprise a keyboard, a touch operated screen or a foot-operated pedal. Desirably, the computer is further provided with at least one of a keyboard and a touch operated screen.

The surgical procedure may be the orthopaedic implantation of a prosthesis, for example the implantation of a knee prosthesis between a tibia and a femur of the patient. In such a procedure there is included resection of a top end of the tibia of the patient. In this case a milestone step in the surgical procedure may be measurement of a gap between a top surface of the resected tibia and at least one of the femoral condyles. Such a gap may be measured with the aid of the tensor of the above first aspect of the present invention.

The gap may be measured with the knee in flexion and/or in extension, and preferably with the knee in flexion and also with the knee in extension.

Another milestone step in the surgical procedure may be the measurement of the tensions in the tibial and fibular collateral ligaments with the patient's leg substantially aligned to the Maquet line.

Thus the at least one surgical measuring device may be at least one of the tensor of the above first aspect and the alignment guide of the above second aspect. In a preferred arrangement, both surgical measuring devices will be used.

Also provided in accordance with the invention is a computer programmed with a computer program for automatically guiding a surgeon through a surgical procedure which includes a number of steps including at least one milestone step and at least one non-milestone step and for use with a computer having an output screen and an input device and connected to at least one surgical measuring instrument adapted to provide an output signal or signals representative of a measurement made by the surgeon following a respective milestone step of the surgical procedure and capable of interacting with the computer program, the computer program being arranged so as to display a plurality of screen displays on the output screen in succession in response to input actions entered on the input device, the plurality of screen displays including at least one milestone screen display corresponding to a respective milestone step of the surgical procedure, so as to permit display of a next succeeding screen display on the output screen upon entry of a corresponding input action on the input device following completion by the surgeon of a non-milestone step of the surgical procedure but so as to block the display of the next screen display following completion by the surgeon of a milestone step unless the output signal or signals from the respective surgical measuring instrument corresponds or correspond to a respective predetermined value or values.

The present invention will now be described, by way of example, with reference to the accompanying drawings, in which:
- Figure 1: is a perspective view of a tensor in accordance with one embodiment of the present invention;
- Figure 2: is a diagrammatic illustration of the correct alignment of a leg of a human patient part way through a surgical operation to implant a knee prosthesis;
- Figure 3: is a corresponding illustration showing an incorrect alignment of the leg;
- Figure 4: is a front schematic view of the knee of the patient of Figure 2;
- Figure 5: is a perspective view of an angular alignment instrument for use in the course of implantation of a knee prosthesis into a knee of a patient;
- Figure 6: is a perspective view of a pair of dividers for use by a left-handed surgeon during the implantation of a knee prosthesis into a knee of a patient;
- Figure 7: is a longitudinal cross-section through a similar pair of dividers for use by a right-handed surgeon; and
- Figure 8: is a top plan view of the pair of dividers of Figure 7.

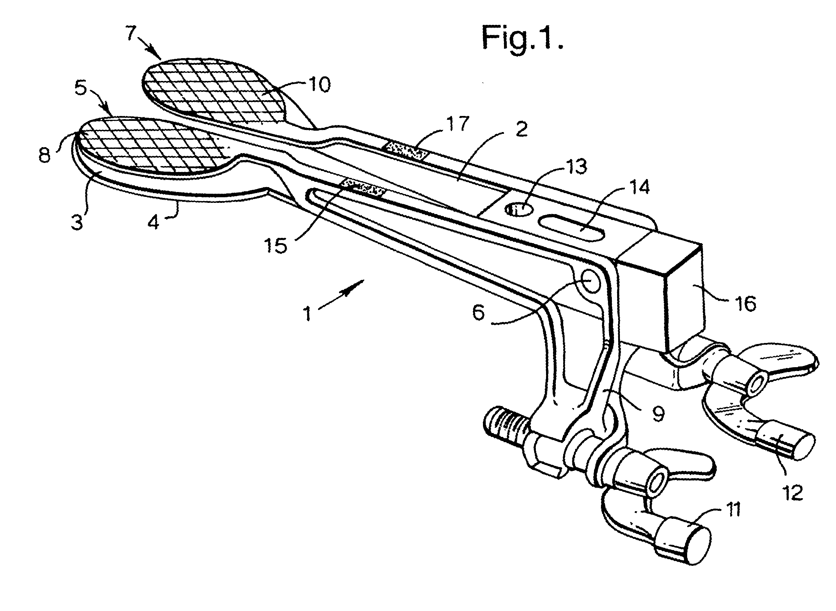

Figure 1 illustrates a tensor 1 for use in the course of a surgical procedure for implantation of a knee prosthesis into a patient, more particularly for measuring the tension in the medial ligaments of the patient's knee following resection of a top portion of the patient's tibia.

Tensor 1 comprises a body 2 integrally formed with a lower tibial paddle 3 having an undersurface 4 for contact with the resected top surface of the patient's tibia. A first upper femoral paddle member 5 is pivotally mounted on one side of body 2 by means of pivot pin 6. A second upper femoral paddle member 7 is mounted on the other side of body by means of a further pivot pin (not shown). First upper paddle member 5 has an upper surface 8 for contact with one of the femoral condyles and is L-shaped in side view with a downwardly extending rear leg 9. Second upper femoral paddle member 7 is similarly shaped and has an upper surface 10 for contact with the other femoral condyle. The front end of first upper femoral paddle member 5 can be raised and lowered relative to the lower tibial paddle member 3 by means of an adjustment device 11 in the form of a compression screw. A similar adjustment device 12 is provided for moving the second upper femoral paddle member 7 relative to the lower paddle member 3.

Body 2 is formed with a bore 13 for passage of an ankle alignment device and also with a slot 14 for cooperation with an angular alignment sensor mechanism (described hereinafter) for determining the position of the Maquet line.

First femoral upper paddle member 5 is provided with a force sensor 15 data from which is collected by suitable electronics contained within a housing 16 at the rear end of the body 2. In addition, second femoral upper paddle member 7 is provided with a force sensor 17, data from which is also connected to the electronics in housing 16. These electronics enable data relating to the forces measured by force sensors 15 and 17 to be transmitted to a computer where, by means of a computer program, they may be suitably manipulated to be stored and/or to allow the data to be displayed to the surgeon. Calculations based on the data may also be performed by the computer program. The data will be transmitted to the computer by any suitable means. Wires (not shown) may connect the tensor to the computer. Alternatively, the information may be transmitted wirelessly.

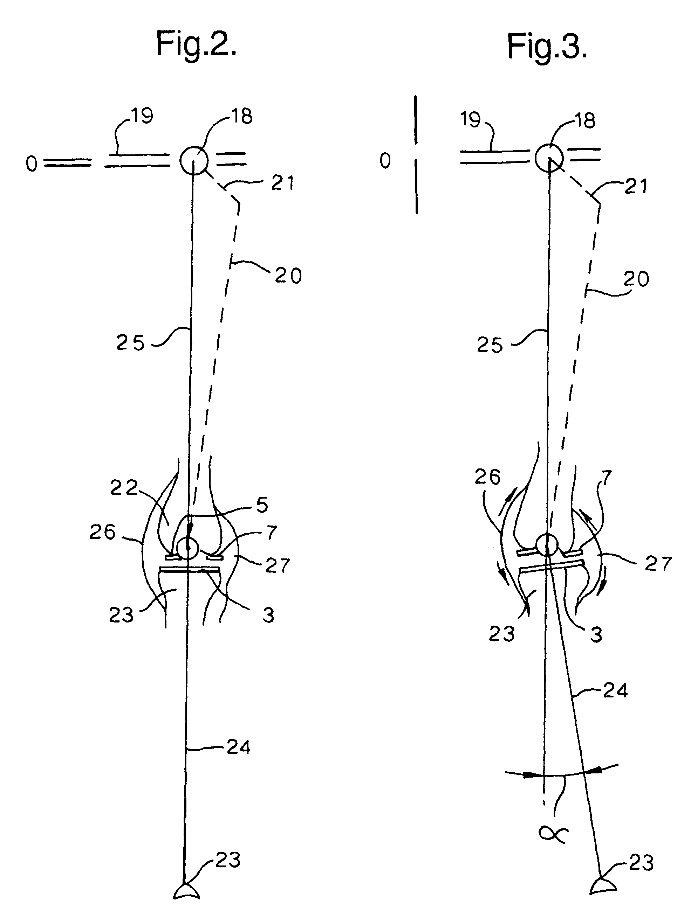

Figure 2 is a diagrammatic front view of the left leg of a patient undergoing implantation of a knee prosthesis and illustrates diagrammatically the correct alignment of the patient's leg with the tensor 1 of Figure 1 in place in the gap between the top resected surface of the patient's tibia and the femoral condyles. In this case the tensions in the medial and lateral ligaments are substantially equal and the leg is appropriately aligned.

Reference numeral 18 indicates the patient's femoral head, the position of which has been located using a pelvic bar 19 as will be described hereinafter, while reference numerals 20 and 21 indicate the axis of the patient's femur 22 and of its femoral neck respectively.

As illustrated, the top end of the patient's tibia 23 has already been resected along a plane at right angles to the axis of the Maquet line, i.e. an imaginary line drawn through the centre of the ankle, knee and hip joints. An ankle alignment rod 24 is inserted in the bore 13 of tensor 1 in alignment with the patient's ankle 23, while a telescopic sliding rod 25 is inserted in slot 14 to indicate the Maquet line. The surgeon can determine when the alignment of the leg is correct by placing the lower surface 4 of the lower tibial paddle member 3 in contact with the resected top surface of the patient's tibia 23 and adjusting the adjustment devices 11 and 12 so as to cause the upper surfaces 8 and 10 to contact the femoral condyles of the patient's femur 22 and put the tibial collateral ligament 26 and the fibular collateral lateral ligament 27 under tension. If the rods 24 and 25 are aligned one with another and if the forces in the ligaments 26 and 27, as measured by the force sensors 15 and 17, are substantially equal, then the surgeon knows that the alignment is correct and that he can proceed to the next step of the surgical procedure. Thus, at this stage of the surgical procedure, the surgeon knows that the centre of the knee joint and the centre of the ankle joint are in alignment and that the tibial cut is correct.

On the other hand, if the alignment is incorrect, as illustrated in Figure 3, so that the tension in the fibular collateral ligament 27 is, for example, greater than in the tibial collateral ligament 26 and so that the rods 24 and 25 are not properly aligned but are at an angle α to one another, then it is necessary for the surgeon to undertake remedial action before proceeding to the next step of the surgical procedure. (In Figure 3 the magnitude of the angle α has been exaggerated somewhat for clarity). This remedial action can take the form of a release of the tibial collateral ligament 26 or of the fibular collateral ligament 27 or both.

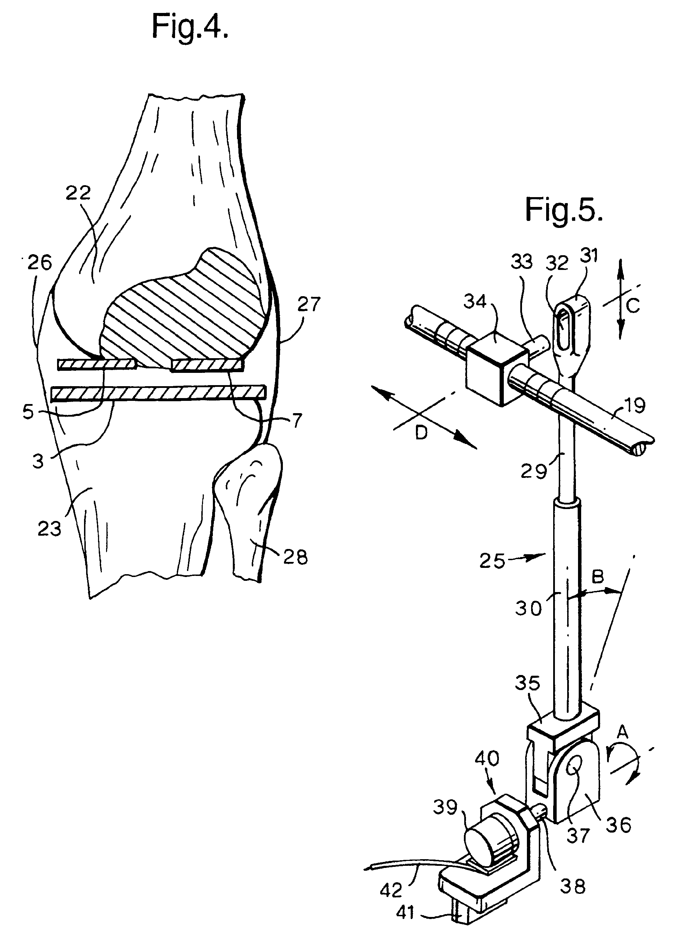

Figure 4 is an enlarged view of part of Figure 2 and shows the use of the tensor 1 in more detail. The patient's fibula 28 is visible in Figure 4.

Figure 5 illustrates in more detail the telescopic sliding rod 25 of Figures 2 and 3. This includes an upper sliding rod 29 slidably received in a lower rod 30. At the upper end of upper sliding rod 29 is an eye portion 31 having an elliptical slot 32 formed therein for reception of a spigot 33 on a sliding pivot block 34 mounted on pelvic bar 19.

The lower end of lower rod 30 is joined to a pivot block 35 which is pivotally mounted between the jaws of a further U-shaped block 36 for pivotal movement about a pivot pin 37. U-shaped block 36 is mounted on a shaft 38 of a potentiometer 39 which is carried on a mounting bracket 40 provided on its underside with a lug 41 sized and shaped to fit in slot 14 of tensor 1. Shaft 38 acts as an axis about which the tensioner can rotate as illustrated by arrow A. If rod 24, which is engaged in bore 13 of tensor 1 is properly aligned with rod 25 as shown in Figure 2, then the output voltage from potentiometer 39 will signal this to the computer by suitable means such as via the sterile connection 42. However, if rod 24 and rod 25 are instead misaligned, as shown in Figure 3, then potentiometer shaft 38 will have been rotated through an angle α and the corresponding output voltage from the potentiometer will indicate this fact and the magnitude of angle α to the computer.

Pivot pin 37 allows pivoting, as indicated by arrow "B" in an anterior-posterior direction. In one arrangement, a potentiometer may also be associated with pivot pin 37 to signal movement in the anterior-posterior direction.

Arrow "C" indicates how vertical adjustment is possible for reduction of restraint, while arrow "D" indicates adjustment along the pelvic bar for location of the femoral head centre.

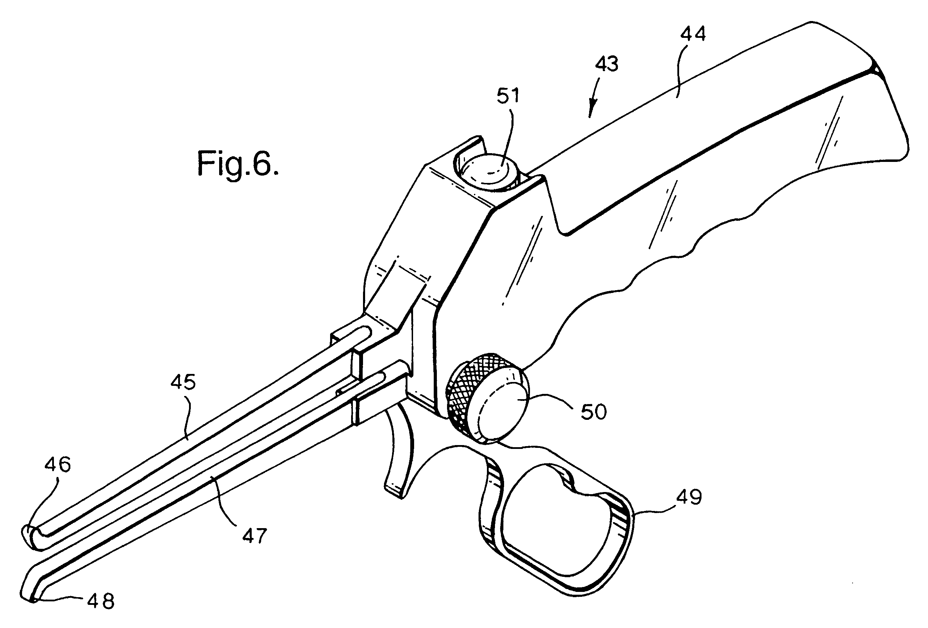

In Figure 6 there is illustrated an electro-mechanical pair of dividers 43, or callipers, designed to use a means for gauging distances and selection of a most suitable thickness of the tibial implant of the knee prosthesis in conjunction with software to be described hereinafter. Suitable means for gauging distances include potentiometers, magnetoresistive devices or the like.

The dividers 43 as illustrated in Figure 6 are for use by a left-handed surgeon during implantation of a knee prosthesis and include a handle 44 from the front end of which projects a fixed arm 45 with an upturned tip 46. A movable arm 47 having a downturned tip 48 is integrally formed with a rearward grip 49 and can be pivoted relative to handle 44 about a pivot pin (not shown) provided with a locking knob 50. Reference numeral 51 indicates a switch. It will be understood that the dividers may be arranged such that the fixed arm may be the lower arm and the upper arm may be movable. Alternatively, both arms may be movable.

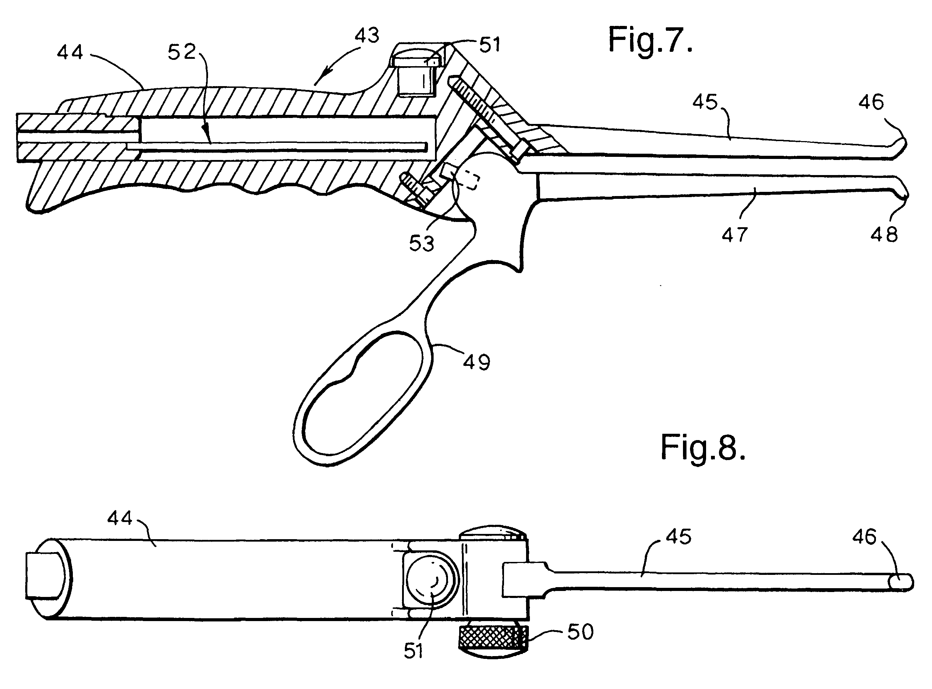

Figures 7 and 8 show a corresponding pair of dividers for use by a right-handed surgeon. The same reference numerals are used in Figures 7 and 8 to indicate like parts to those of the pair of dividers of Figure 6. A printed circuit board 52 is mounted inside handle 44 and a magnet 53 is fitted to movable arm 47 for interaction with a sensor or sensors, which may be potentiometer or magnetoresistive, mounted within the handle 44 to ensure instant interchangeability. Dividers 43 are connected to the computer by a suitable means. The connection means may be wireless or may be via a sterile wire.

Instead of providing sterile connections for connecting the surgical instruments of the invention to the computer, any other known means of communication between an input device and a computer can alternatively be provided, for example an infra-red emitter, an ultra-violet light emitter, or a radio transmitter on the instrument which send a signal to an infra-red receiver, an ultra-violet light receiver, or a radio receiver positioned where it can readily receive the output signal from the instrument, for example on the computer itself or on the ceiling or a wall of the operating theatre.

The surgical procedure to be followed may be, for example, that set out for the Protek FS knee in a brochure published by its manufacturers. However, the invention can readily be applied with suitable adjusted software to any surgical procedure involving measurement of physical dimensions.

The computer can be a conventional computer with an appropriate integral or separate display screen, for example, a laptop computer. The computer is arranged conveniently in the operating room in the view of the surgeon and is provided with a foot switch as an input device. This foot switch could be available to the surgeon, the scrub nurse, an assistant, or anyone else in the room.

The computer software is written in such a format as to present in turn a series of display screens, each relating to a corresponding step of the surgical procedure. The software is further designed so that the surgeon can run through the operation that he is going to carry out in advance of the actual operation to implant the knee prosthesis. With the aid of the foot switch the surgeon can move backwards or forwards from screen to screen, subject to the constraints mentioned hereinafter. The person operating the computer can alternatively use the computer keyboard to make various entries on the screen. The computer may alternatively be operated by other means such as by being voice activated.

The initial screens describing, for example, the non-milestone steps of the surgical procedure (such as incision, dislocation of the patella, and the like) need only be described briefly. They can further be illustrated by suitable diagrams or photographs. These screens act as a prompt for a surgeon who is unsure of the operation, perhaps as a result of being called upon to perform it at infrequent intervals. The program can be arranged so that, by use of the foot pedal, an indication, such as an asterisk, can be placed by any particular entry which would in turn enable the surgeon to make written additions related to this step in the final printed record of the surgical procedure.

When the surgeon gets to the end of the step or steps to which a particular screen relates (for example, having completed the incision and dislocated the patella) and indicates this by pressing the foot pedal or the return key on the computer keyboard, the computer program can put up a message on the computer screen to ask if the steps on the screen have been satisfactorily completed. If an affirmative answer is given by use of the input device, the computer program then causes a display screen appropriate to the next step in the operation to be displayed on the output screen. At the end of the procedure the contents of all screens to which the surgeon had responded positively, or a brief summary thereof, can then be printed so as to constitute an operative note.

An optional facility is to have a clock running as part of the computer program during the operation which would record the time at which each screen was changed. This would record the duration of the procedure which might be helpful for surgeons and trainees.

Certain screens not only set out the steps to be accomplished but they also show various key factors of importance to correct performance of the operation, such as distances, and tensions in the tibial and fibular collateral ligaments when the surgical procedure is the implantation of a knee prosthesis. This facility can be used to provide a series of prompts to the surgeon.

Apart from the surgical instruments connected or otherwise in communication with the computer the basic instruments used by the surgeon can be those conventionally used for knee replacement operations. The program can be arranged to work with IM or EM alignment.

The use of the instruments shown in Figures 1 to 8 the drawings may be illustrated by steps in the FS procedure. Before forming the extension gap, the femur 22 is cut anteriorly and posteriorly and the tibia 23 proximally as follows. In flexion the relevant display screen is arranged to show the bone suitably cut and to indicate where the tensor 1 is to be placed. Once these steps have been taken, the surgeon can then click the foot switch and carry on to the next step on that screen.

The next step consists of positioning the tensor 1 in the opening with the surface 4 in contact with the resected top surface of the tibia 23. Then the surgeon operates the compression screws 11 and 12 until the surfaces 8 and 10 of the upper condylar paddle members 5 and 7 contact the respective condyles. As the compression screws 11 and 12 are operated, the force sensors 15 and 17 generate an output signal dependent upon the respective tensions produced in the tibial and fibular collateral ligaments. As this is done the tensions measured via the bending moment of the paddle members 5 and 7 by the force sensors 15 and 17 are displayed on the output screen in a diagram medially and laterally. When these tensions each lie in a range of acceptable values, the appropriate values are displayed in green. On the other hand, if the tensions in the collateral ligaments lie outside that range, i.e. are too small or too great, then the values are displayed in red. The surgeon has the option of achieving the correct tension only medially, or medially and laterally, in which latter case the gap between the condyles and the resected surface of the tibia would be trapezoidal rather that rectangular.

Once the correct tensions in the collateral ligaments have been achieved, the surgeon passes on to the next step. The computer program records what tensions had been achieved. Unless and until the correct tensions have been achieved the computer program can be arranged to prevent the surgeon from moving on to the next display screen.

In the following step the surgeon uses the dividers 43 to measure the gap either medially or medially and laterally. This gap is displayed by the computer program on the output screen as increasing thicknesses of tibial component. Once again when this has been done and the surgeon passes to the next step, the computer program records the tibial thickness required.

If desired, the surgeon at this stage can use the computer to measure the anterior-posterior (AP) and medial-lateral (ML) dimensions of the cut top of the tibia 23. In this case the surgeon now knows the thickness and size of tibial component to be used.

The program then prompts the surgeon to respond whether he had completed the steps outlined on this display screen satisfactorily. If the surgeon response positively, the time lapse in the operation can be recorded and the tensions and distances measured recorded in the final operative note.

The next display screen shows the extension gap with the tensor 1 in place and the alignment bar 25 going up towards the hip 18. If, for example, the position of the hip 9 has been measured by an X-ray of the pelvis pre-operatively with an appropriate bar and found to be 9 cm from the symphysis pubis, the surgeon opens the dividers 43 to the appropriate distance (9 cm) and places one limb of the dividers 43 on the symphysis found by palpation. The two paddle members 5 and 7 of tensor 1 can then be opened until the bar 25 on the tensor touched the second limb of the dividers 25. If desired, the computer program can be arranged to display this situation on the display screen by showing the alignment bar 25 sweeping either from the medial or the lateral side towards the correct position.

As the two paddle members 5 and 7 of the tensor 1 are opened the tension in the collateral ligaments on each side of the knee are displayed on the output screen. If the alignment bar 25 arrives at the correct position with tension measurements in the desired range so that the output screen indicated that these are correct (i.e. by indicating them in green) on both sides, the surgeon has now satisfactorily aligned and stabilised the knee. If correct alignment can only be achieved with one side still showing red on the output screen, the computer program can be arranged to calculate which side of the knee needs a soft tissue release and to indicate that on the display screen. The surgeon then carries out the release and repeats the alignment procedure.

Unless and until the tensions in the collateral ligaments are correct the computer program does not display the output signal from potentiometer 39 indicating that alignment has been achieved together with the desired values for those tensions. In addition the program will not let the surgeon move on to the next display screen.

When correct alignment has been obtained, the dividers 43 are closed until they measure whatever gap has been obtained in flexion. The computer program calculates this value and indicates to the surgeon when the right separation of the dividers 43 has been achieved.

The surgeon then uses the dividers 43 to mark upwards from the tibia 23 onto the medial and lateral femoral condyles of the femur 22 to define the attitude and proximal distal level of the distal femoral cut. Once this has been done, all the relevant information can be recorded together with the time elapsed, as for the flexion step.

The rest of the operation can now proceed with the computer program arranged to present a series of display screens which do not really require any interactive element, although interactive elements could be included, if desired, in order to ensure that the surgeon records full details of the operation.

When the surgeon has closed the skin, the computer program provides a final display screen which, upon completion by the surgeon, gives a total tourniquet time to the computer.

The computer can now be connected to a printer and to a bar coder. The bar coder is used to input the details of the prostheses used and the printer can then be used to print out an operative note. Such an operative note can be in very small print or in abbreviated form. The note is conveniently produced on conventional hospital paper and printed in the operating theatre so that it can go with the patient into the recovery room. Any small print used should still be sufficiently large to be legible to the nurses in the recovery room.

The computer program can, if desired, include a feature whereby the surgeon can put asterisks by some or all of the steps of the surgical procedure. In this case the computer can be arranged to present those steps at the foot of the operative note so as to permit the surgeon to write any desired remarks directly ono the notes. Such remarks can be typed via the computer keyboard or handwritten in the appropriate footnotes. The surgeon can in this way have an automatically produced operative note containing far more information than even the best handwritten notes do according to conventional practices.

Either at the beginning of the operation or at the end thereof, the computer program includes a display screen where administrative data such as the patient's name, the patient's record number, operative date, the name of the surgeon, whether the operation has been upon the left knee or the right knee, and the like, can be entered.

It is also possible for the computer program to record at least some of the patient's physiological data, such as blood pressure, heart rate, and the like. Also the details of the anaesthetic procedures used could be recorded by the computer program.

The computer program described above can be written in any appropriate computer language. It will be apparent to a competent computer programmer how such a program should be written.

Although the computer program has been described in relation to a knee replacement operation using a particular type of knee prosthesis, the general teachings of the invention can be applied to implantation of any other type of knee prosthesis or indeed to any other orthopaedic or other surgical procedure in which success requires careful measurement of a distance or of some other property, such as the tension in a tendon or the like.

## Claims

**1.** A tensor for use during the surgical implantation of a knee prosthesis including a tibial component and at least one femoral component, the tensor comprising:
a lower paddle having an under surface for contacting an upper surface of a resected tibia of the patient;
a first upper paddle movable towards and away from the lower paddle and having an upper surface for contacting a first one of the condyles of a corresponding femur of the patient;
a second upper paddle movable towards and away from the lower paddle and having an upper surface for contacting the other one of the condyles of the femur of the patient;
first adjustment means for moving the first upper paddle towards and away from the lower paddle;
second adjustment means for moving the second upper paddle towards and away from the lower paddle;
a first force sensor means associated with the first upper paddle which in use will measure a first force exerted by the first upper paddle against the first one of the condyles when the under surface of the lower paddle is in contact with the resected upper surface of the tibia of the patient and the first upper paddle is moved progressively away from the lower paddle into contact with the first one of the condyles;
a second load sensor means associated with the second upper paddle which in use will measure a second force exerted by the second upper paddle against the other one of the condyles when the under surface of the lower paddle is in contact with the resected upper surface of the tibia of the patient and the second upper paddle is moved progressively away from the lower paddle into contact with the other one of the condyles; and
signal output means for receiving signals from the first and second load sensor means indicative of the first and second forces respectively and for transmitting at least one output signal derived therefrom.

**2.** An alignment device for use during the surgical implantation of a knee prosthesis to aid in identifying the Maquet line, the device comprising:
first mounting means for location in the vicinity of a resected upper surface of a corresponding tibia of the patient;
a first pivot member mounted in the first mounting means so as to permit pivotal movement about a medial-lateral axis relative to the first mounting means;
detector means for detecting the magnitude of medial-lateral pivotal movement of the first pivot member;
second mounting means mounted to the pivot member;
elongate indicator means pivotally mounted at a lower end thereof in the second mounting means so as to permit pivotal movement about an anterior-posterior axis relative to the second mounting means, the elongate indicator means further having an upper end for positioning adjacent a hip of a patient; and
output means for producing an output signal from the detector means corresponding to the angular position of the first pivot member for transmitting said signal.

**3.** A computer program element comprising computer code means which when loaded on a computer will be adapted:
to receive output signals from at least one of a tensor of the above first aspect and an alignment device of the above second aspect;
to display information relating to the received signals; and
to receive input from a user.

**4.** The computer program element according to Claim 3 wherein the element will also compare information derived from the output signals with stored data such that information relating to corrections which may be required can be displayed.

**5.** The computer program element according to Claim 3 or 4 additionally providing means to enable the output signals and the input from the user to be recorded.

**6.** The computer program element according to any one of Claims 3 additionally providing means to record the time at which the output signals and the input from the user are received.

**7.** A computer program comprising the computer program element of any one of Claims 3 to 6.

**8.** The computer program according to Claim 7 additionally including means to enable the user to input additional notes.

**9.** The computer program according to Claim 8 where in the input of additional notes may be recordal of voice.

**10.** The computer program according to any one of Claims 7 to 9 additionally enabling the display of information relating to the procedure to be carried out.

**11.** The computer program according to any one of Claims 7 to 10 configured such that the user interface is provided as a plurality of screens.

**12.** The computer program according to Claim 11 wherein each screen includes an interactive prompt to ask the user to confirm that the steps referred to on the screen had been successfully completed.

**13.** A computer readable medium having the computer program of any one of Claims 7 to 12 recorded thereon.

**14.** A kit comprising the tensor of Claim 1, the alignment guide of Claim 2 and the computer readable medium of Claim 13.

**15.** The kit according to Claim 14 additionally including instructions.

**16.** An automatic method of guiding a surgeon through a surgical procedure which includes a number of steps including at least one milestone step and at least one non-milestone step, the method comprising:
providing a computer having an output screen and an input device and loaded with a computer program arranged to display a plurality of screen displays on the output screen in response to input actions entered on the input device;
providing at least one surgical measuring instrument adapted to provide an output signal or signals representative of a measurement made by the surgeon following a respective milestone step of the surgical procedure and capable of interacting with the computer program; and
displaying on the output screen in turn a plurality of screen displays in succession in response to input actions entered on the input device, the plurality of screen displays including at least one milestone screen display corresponding to a respective milestone step of the surgical procedure;
wherein the computer program is arranged (i) to permit display of a next succeeding screen display on the output screen upon entry of a corresponding input action following completion by the surgeon of a non-milestone step of the surgical procedure and (ii) to block the display of the next screen display following completion by the surgeon of a milestone step unless the output signal or signals from the respective surgical measuring instrument corresponds or correspond to a respective predetermined value or values.

**17.** An automatic method according to Claim 16 wherein the computer program is be arranged to record a value or values corresponding to an output signal or signals measured by the surgical measuring instrument at the completion of the or each milestone step of the surgical procedure.

**18.** An automatic method according to Claim 16 or 17 wherein the computer program is arranged to record a time at which at least one preselected input action is entered on the input device.

**19.** An automatic method according to any one of Claims 16 to 18 wherein the computer program is arranged to record one or more physiological characteristics of the patient during the course of the surgical procedure.

**20.** An automatic method according to any one of Claims 16 to 19 wherein the computer program is arranged to record details of the patient prior to the commencement of the surgical procedure.

**21.** An automatic method according to any one of Claims 16 to 20 wherein the surgical procedure is the orthopaedic implantation of a prosthesis.

**22.** An automatic method according to Claim 21 wherein the prosthesis is a knee prosthesis which is to be located between a tibia and a femur of the patient.

**23.** An automatic method according to Claim 22 wherein a milestone step in the surgical procedure is the measurement of a gap between a top surface of a resected tibia and at least one of the femoral condyles.

**24.** An automatic method according to Claim 23 wherein the gap is measured with the aid of the tensor of Claim 1.

**25.** An automatic method according to Claim 23 or 24 wherein the gap is measured with the knee in flexion and/or in extension.

**26.** An automatic method according to any one of Claims 16 to 25 wherein another milestone step in the surgical procedure is the measurement of the tensions in the tibial and fibular collateral ligaments with the patient's leg substantially aligned to the Maquet line.

**26.** An automatic method according to Claim 26 wherein the measurement is carried out using the alignment device of Claim 2.

**27.** A computer programmed with a computer program for automatically guiding a surgeon through a surgical procedure which includes a number of steps including at least one milestone step and at least one non-milestone step and for use with a computer having an output screen and an input device and connected to at least one surgical measuring instrument adapted to provide an output signal or signals representative of a measurement made by the surgeon following a respective milestone step of the surgical procedure and capable of interacting with the computer program, the computer program being arranged so as to display a plurality of screen displays on the output screen in succession in response to input actions entered on the input device, the plurality of screen displays including at least one milestone screen display corresponding to a respective milestone step of the surgical procedure, so as to permit display of a next succeeding screen display on the output screen upon entry of a corresponding input action on the input device following completion by the surgeon of a non-milestone step of the surgical procedure but so as to block the display of the next screen display following completion by the surgeon of a milestone step unless the output signal or signals from the respective surgical measuring instrument corresponds or correspond to a respective predetermined value or values.
